# EUROPÄISCHE PATENTSCHRIFT

(11) **EP 3 313 319 B1**
(45) Veröffentlichungstag und Bekanntmachung des Hinweises auf die Patenterteilung: **22.06.2022**
(21) Anmeldenummer: 16728309.2
(22) Anmeldetag: 09.06.2016
(51) Int. Cl.: A61B 50/30, A61B 50/00

(54) **STERILCONTAINER MIT EINER MEHRZAHL VON ZENTRAL BETÄTIGBAREN VERRIEGELUNGSELEMENTEN**
STERILE CONTAINER WITH A MULTITUDE OF CENTRALLY ACTUABLE LOCKING ELEMENTS
RÉCIPIENT STÉRILE COMPORTANT UNE PLURALITÉ D'ÉLÉMENTS DE VERROUILLAGE POUVANT ÊTRE ACTIONNÉS DE FAÇON CENTRALE

(30) Priorität: 29.06.2015 DE 102015110419
(43) Veröffentlichungstag der Anmeldung: 02.05.2018
(73) Patentinhaber: Aesculap AG, 78532 Tuttlingen (DE)
(72) Erfinder: WEISSHAUPT, Dieter, 78194 Immendingen (DE)
(74) Vertreter: Winter, Brandl - Partnerschaft mbB
(86) Internationale Anmeldenummer: PCT/EP2016/063204
(87) Internationale Veröffentlichungsnummer: WO 2017/001166

(56) Entgegenhaltungen:
- WO-A2-2008/078169
- CN-B- 101 121 031
- DE-U1-202008 009 661
- US-A- 5 641 065

## Beschreibung

Die vorliegende Erfindung betrifft einen Sterilcontainer mit einer Mehrzahl von zentral betätigbaren Verriegelungselementen und insbesondere einen Sterilcontainer mit einer Mehrzahl von über ein Betätigungsband durch mindestens einen gemeinsamen Betätigungshebel betätigbaren Verriegelungselementen, wobei die Anzahl an Betätigungselementen kleiner ist als die Anzahl an durch sie betätigbaren Verriegelungselementen.

### Hintergrund der Erfindung

Bei der überwiegenden Mehrheit klinischer Handlungen und Operationen ist die Gewährleistung der Sterilität der verwendeten Instrumente und / oder sonstiger Hilfsmittel unerlässlich. Aus diesem Grund werden Sterilcontainer /-behälter (auch als Sterilisationscontainer /-behälter bezeichnet) verwendet, welche beispielsweise mit medizinischen Instrumenten bestückt / befüllt werden.

Daraufhin wird der Sterilisationscontainer im bestückten / befüllten Zustand beispielsweise in einem Autoklaven für eine vorherbestimmte Zeitdauer auf eine vorherbestimmte Sterilisationstemperatur erhitzt, bis etwaige an den medizinischen Instrumenten anhaftende Mikroorganismen abgetötet sind.

Bei dem Sterilisationsprozess, beispielsweise in einem Autoklaven, kommt es zur Kondensatbildung im Inneren des Sterilcontainers. Um dieses Kondensat aus dem Sterilcontainer zu entfernen wird bei Containersystemen mit aktiver Kondensatausleitung der Druckunterschied zwischen dem Überdruck in dem Sterilcontainer und dem Unterdruck in der Sterilsationskammer dazu verwendet, das Kondensat abzuführen.

Herkömmliche Sterilcontainer weisen in der Regel einen an eine Containerwanne ansetzbaren Deckel oder eine Tür sowie ein oder zwei Verriegelungselemente auf, mit dem / denen der Deckel oder die Tür am Sterilcontainer geschlossen gehalten und verriegelt wird. Bei derartigen herkömmlichen Sterilcontainern kann es bei der aktiven Kondensatausleitung aufgrund des Druckunterschieds zwischen dem Inneren des Sterilcontainers und der Sterilisationskammer zu Deformationen der Containerwanne bzw. des Containerkörpers und des Deckels des Containers kommen, durch welche der Überdruck entweichen kann bzw. ein Druckausgleich stattfinden kann. Solche herkömmlichen Sterilcontainer sind beispielsweise in WO 2008 / 078 169 A2, US 5 641 065 A, DE 20 2008 009 661 U1 oder CN 101 121 031 B gezeigt.

Derartige Deformationen jedoch führen dazu, dass in dem Sterilcontainer nach einem derartigen unkontrollierten Druckausgleich nicht mehr genügend Druck zurückbleibt, um alles in dem Sterilcontainer enthaltene Kondensat erfolgreich auszubringen. Dies führt zu einer unerwünschten Restfeuchtigkeit im Innern des Sterilcontainers.

### Zusammenfassung

Der vorliegenden Erfindung liegt die Aufgabe zugrunde, einen Sterilcontainer mit aktiver Kondensatausleitung bereitzustellen, bei dem Deformationen des Sterilcontainers aufgrund von Druckunterschieden weitestgehend vermieden werden, bzw. zu keinem Druckverlust führen.

Diese Aufgabe wird gelöst durch den Sterilcontainer nach Anspruch 1. Vorteilhafte Weiterbildungen sind Gegenstand der Unteransprüche.

Ein erfindungsgemäßer Sterilcontainer mit aktiver Kondensatausleitung weist eine Mehrzahl von betätigbaren Verriegelungselementen auf, welche über (mindestens) ein Betätigungsband durch (mindestens) einen gemeinsamen Betätigungshebel betätigbar sind bzw. betätigt werden. D.h. mehrere (Containerumfangs-beabstandete) Verriegelungselemente oder Verriegelungsmittel sind (funktionell) zu einem Verriegelungsmechanismus zusammengefasst, der mittels einer (manuellen) Betätigungseinrichtung bzw. einem Betätigungsmittel (unhabhängig zu ggf. noch weiteren Verriegelungsmechanismen diesen Aufbaus) gemeinsam aktuierbar ist.

Der (mindestens eine) Betätigungshebel ist dabei derart ausgelegt, dass er einen Exzenterpunkt überschreitet, wenn er in eine Verriegelungsposition gebracht wird, wodurch ein unbeabsichtigtes und selbsttätiges Aufspringen des mindestens einen Betätigungshebels in eine Öffnungsposition verhindert wird (selbsthemmend).

Der Betätigungsaufwand bleibt somit gegenüber dem bekannten Stand der Technik der Gleiche, wohingegen die Schließsicherheit aufgrund der Anordnung mehrerer gemeinsam/zentral durch ein Betätigungsmittel betätigter Verriegelungsmittel verbessert wird.

Die Kernidee liegt demzufolge darin, dass die mehreren, voneinander Containerumfangs-beabstandeten oder Öffnungsumfangs-beabstandeten Verriegelungselemente eine stabile, weil mehrpunktige, Verbindung zwischen dem Containerdeckel/Tür und der Containerwanne des Sterilcontainers bieten, was die Widerstandskraft des Sterilcontainers gegenüber Deformationen erhöht. Hierdurch werden Leckagen aufgrund von während des Sterilsationsprozesses auftretender Deformationen vermieden und der Druckausgleich zwischen dem Inneren des Sterilcontainers und der Sterilisationskammer kann kontrolliert und effizient zur Ausbringung des in dem Sterilisationscontainer anfallenden Kondensats genutzt werden.

In anderen Worten ausgedrückt, befinden sich an der Containerwanne und am Containerdeckel eine Mehrzahl fest daran angeordneter Verriegelungselementepaare, von denen zumindest ein Verriegelungselement jedes Verriegelungselementepaars beweglich an der Containerwanne oder am Containerdeckel, unentfernbar gehalten ist und mit einem anderen Verriegelungselement desselben Paares in Riegeleingriff bringbar ist. Das vorzugsweise biegeflexible, zumindest eine Betätigungsband hat daran montierte/ausgebildete Eingriffsglieder/Eingriffsabschnitte, wie Vor- /Rücksprünge, Bohrungen, etc. die selbst keine Verriegelungsfunktion haben, d.h. nicht unmittelbar im Verriegelungskraftfluss liegen, aber das bewegliche Verriegelungselement des jeweiligen Verriegelungselementepaars für ein Ver- /Entriegeln betätigen. Damit kann das Betätigungsband nur bezüglich der Betätigungsfunktion dimensioniert sein, da es per se keine Verriegelungskräfte aufnehmen muss.

Gemäß einer vorteilhaften Ausführungsform weist der Sterilcontainer zwei Betätigungshebel auf, welche jeweils mit einer Mehrzahl (mehr als eins) von Verriegelungselementen gekoppelt sind und welche gemeinsam eine Zweihandbedienung zum Öffnen und Schließen des Sterilcontainers ermöglichen. Diese zwei Betätigungshebel sind vorzugsweise an einander gegenüberliegenden Seiten des Sterilcontainers angeordnet.

Gemäß einer weiteren vorteilhaften Ausführungsform ist an dem, dem (mindestens einen) Betätigungshebel entgegengesetzten Ende des daran angekoppelten Betätigungsbands ein elastisches Spannungselement, vorzugsweise eine Feder, vorgesehen, das vorzugsweise am Sterilcontainer (Deckel oder Wanne) festgehalten wird. Das Spannungselement dient dazu, beim Öffnen des Deckels des Sterilcontainers bzw. Aufschwenken des Betätigungshebels die Rückführung der Verriegelungselemente in die Öffnungsposition zu unterstützen, welche durch das des Betätigungsband über den (mindestens einen) Betätigungshebel zurückgedrückt werden. Zudem reduziert das Spannungselement das Spiel in dem Verriegelungsmechanismus des Sterilcontainers.

Die Verriegelungselemente können vorzugsweise haken- oder klauenförmige Schwenkelemente, verschiebbare Verriegelungsstifte oder verschiebbare Verriegelungspilze sein. Bei einer Verriegelung durch Schwenkelemente wird durch die Betätigung der/des Betätigungshebels in Verriegelungsposition Zug (oder Druck) auf das daran gekoppelte, sowie längs der Container- oder Deckel/Türumfangs sich erstreckende Betätigungsband aufgebracht, wodurch die an der Containerwanne oder am Deckel gelagerten Schwenkelemente derart verschwenkt werden, dass sie in entsprechende Gegenstücke wie beispielsweise eine Öse, Riegel oder dergleichen Hinterschneidung eingreifen, wodurch der Sterilcontainer verriegelt wird. Werden Verriegelungspilze bzw. Verriegelungsstifte als Verrieglungselemente verwendet, so weist vorteilhafterweise das Betätigungsband mindestens eine keilförmige Kulisse oder Wölbung auf, welche sich, wenn Zug/Druck auf das Betätigungsband aufgebracht wird, entlang der Longitudinalrichtung des Betätigungsbands bewegt, wodurch ein naheliegender Verriegelungsstift /-pilz in einer im Wesentlichen rechtwinklig zu der der Longitudinalrichtung des Betätigungsbands verlaufenden Richtung verschoben wird, bis sich der Verriegelungsstift /-pilz im Eingriff befindet mit einem entsprechenden Gegenstück wie beispielsweise einer Aussparung oder einer Öse.

Gemäß einer anderen vorteilhaften Ausführungsform zeichnet das Betätigungsband die Kontur (Umfang) eines Deckels/einer Tür des Sterilcontainers im Wesentlichen nach. In anderen Worten verläuft das Betätigungsband zumindest abschnittsweise im Wesentlichen entlang der Außenkante oder der Innenkante des Deckels des Sterilcontainers. In noch anderen Worten verläuft das Betätigungsband entlang der inneren, der Containerwanne des Sterilcontainers zugewandten Fläche des Containerdeckels.

Gemäß einer anderen vorteilhaften Ausführungsform ist ein einstückiges Betätigungsband vorgesehen, welches im Wesentlichen den gesamten Umfang des Deckels des Sterilcontainers umläuft. In anderen Worten bildet ein derartiges biegeflexibles Betätigungsband einen (offenen) Ring aus, welcher den Umfang des Deckels des Sterilcontainers umläuft. Das Betätigungsband kann ein Seilzug, ein Gestänge und/oder ein Flachband sein. Alternativ können auch mehrere Betätigungsbänder vorgesehen sein, welche den Umfang des Deckels des Sterilcontainers jeweils für einen definierten Abschnitt umlaufen. Beispielsweise können zwei Betätigungsbänder vorgesehen sein, welche jeweils eine Hälfte des Deckels des Sterilcontainers umlaufen.

Gemäß einer anderen vorteilhaften Ausführungsform ist das Betätigungsband vorzugsweise an den Ecken des Deckels über Lagerungselemente am Sterilcontainer (Wanne oder Deckel) gelagert und/oder geführt. Hierdurch wird eine einfache Betätigung der Betätigungshebel ermöglicht.

Gemäß einer anderen vorteilhaften Ausführungsform dient das Betätigungsband ausschließlich der Betätigung der an das Betätigungsband gekoppelten Verriegelungselemente. Das heißt, das Betätigungsband hat neben dieser Primärfunktion der Betätigung der Verriegelungselemente keine andere Nebenfunktion.

Gemäß einer anderen vorteilhaften Ausführungsform ist auch das Betätigungsband an dem Containerdeckel oder der Containerwanne unlösbar. Hierdurch wird erreicht, dass sich das Betätigungsband auch bei wiederholtem Öffnen und Schließen des Sterilcontainers von diesem nicht löst und daher vor Beschädigungen geschützt ist, wodurch sich die Lebensdauer des Sterilcontainers erhöht.

Gemäß einer anderen vorteilhaften Ausführungsform weisen einzelne Verriegelungselemente jedes Verriegelungselementepaars interne Verriegelungsglieder auf, welche an der Containerwanne oder dem Containerdeckel verschiebbar und / oder verschwenkbar gelagert sind und welche zur Bewegungsbetätigung/Ver-/Entriegeln mit dem Betätigungsband (2) in Wirkeingriff stehen.

Ein jedes Verriegelungselement kann hierbei ein oder mehrere Verriegelungsglieder aufweisen. Unter Wirkeingriff ist hierbei zu verstehen, dass die Verriegelungsglieder jeweils nicht an dem Betätigungsband fixiert sind, sondern lediglich durch dieses Betätigungsband betätigbar sind. In anderen kann über das Betätigungsband auf die Verriegelungsglieder nur betätigend eingewirkt werden, ohne dass Verriegelungsglieder fest mit dem Betätigungsband verbunden sind.

### Figurenbeschreibung

Weitere Vorteile und Merkmale der vorliegenden Erfindung ergeben sich aus der Beschreibung bevorzugter Ausführungsformen unter Bezugnahme auf die Figuren. Hierbei zeigt:
Fig. 1 einen erfindungsgemäßen Sterilcontainer mit zwei Betätigungshebeln und einem in sich geschlossenen Betätigungsband;
Fig. 2 einen erfindungsgemäßen Sterilcontainer mit einem Betätigungshebel und einem in sich geschlossenen Betätigungsband;
Fig. 3 einen erfindungsgemäßen Sterilcontainer mit zwei Betätigungshebeln und zwei Betätigungsbändern;
Fig. 4 die Einfügung der Führung für das Betätigungsband in der Dichtung des Sterilcontainers; und
Fign. 5a-c verschiedene Arten von Verriegelungselementen.

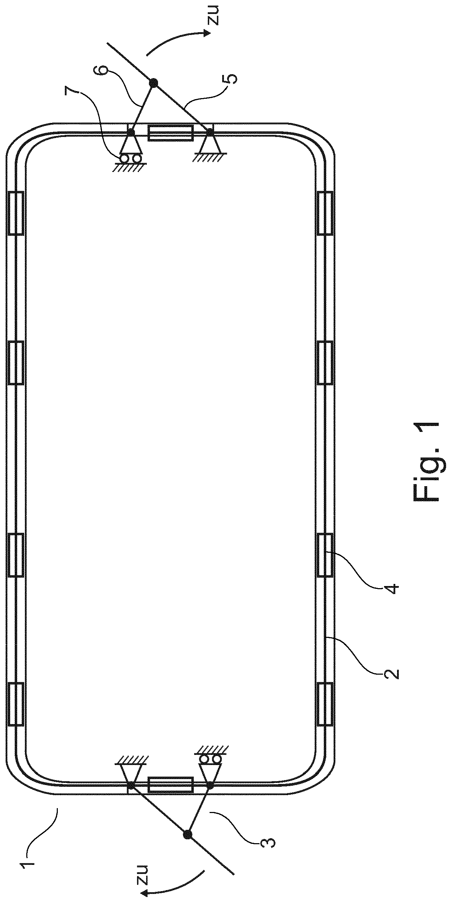

Fig. 1 zeigt einen Sterilcontainer 1 mit (aktiver) Kondensatausleitung mit einer Mehrzahl von über zwei Betätigungsbänder 2 durch zwei gemeinsame Betätigungshebel 3 betätigbaren Verriegelungselementen 4. Auch wenn hier beispielshaft ein Sterilcontainer mit aktiver Kondensatausleitung angeführt ist, kann die Erfindung auch auf einen sonstigen beliebigen Sterilcontainer angewandt werden.

Konkreter ausgedrückt hat der Sterilcontainer 1 eine Containerwanne 1a und einen Containerdeckel 1b, die mittels mindestens einem, vorliegend zwei Verschluss- oder Verriegelungsmechanismen, jeweils bestehend aus (wenigstens) einem Betätigungshebel 3, einem mit dem Betätigungshebel 3 gekoppelten Betätigungsband 2 sowie mehreren mit dem Betätigungsband 2 gekoppelten Verriegelungselementen 4 besteht, miteinander verriegelbar sind. D.h. es sind mehrere, längs des Umfangs des Containerdeckels (oder Tür) beabstandete Verriegelungselemente oder Verriegelungsmittel 4 über ein gemeinsames Betätigungsband 2 als Kraftübertragungsmittel mit jeweils einem Betätigungshebel oder Betätigungsmittel 3 gekoppelt oder wirkgekoppelt.

In dieser besonderen Ausführungsform sind die beiden Betätigungsbänder 2 durch endseitige Überlappen oder durch entsprechende teleskopartige Kopplungsstücke zu einem geschlossenen Ring zusammengefasst, welcher den gesamten Umfang des Deckels des Sterilcontainers 1 umläuft und dessen Konturen im Wesentlichen nachzeichnet. Die beiden Betätigungsbänder 2 befinden sich sowohl beim

Öffnungsvorgang als auch beim Schließ- bzw. Verriegelungsvorgang des Sterilcontainers vorzugsweise durchgehend auf Zug, weswegen alternativ zu den Betätigungsbändern auch Seilzüge, z.B. aus einem Drahtseil, verwendet werden können.

Ein jeder der Betätigungshebel 2 weist zwei Schenkel 5, 6 auf, von denen einer 5 fest mit dem Sterilcontainer oder mit dem einen Betätigungsband 2 endseitig verbunden ist und der Andere 6 beweglich mit dem anderen Betätigungsband 2 endseitig verbunden ist, derart, dass die Betätigungsbänder 2 im Bereich der Anlenkstellen mit den Schenkeln 5 bzw. 6 durch entsprechend Lager 7 am Containerdeckel oder an der Containerwanne gelagert sind, um über die Betätigungshebel 3 längsverschoben werden zu können ohne sich vom Containerdeckel oder von der Containerwanne abzulösen.

Wird einer der Betätigungshebel 3 betätigt bzw. bedient, so bewegt sich der beweglich gelagerte Schenkel des Betätigungshebels 3 und erlaubt somit ein Umklappen des anderen Schenkels des Betätigungshebels, wobei sich die beiden Betätigungsbänder 2 relativ zueinander längsbewegen. Vorteilhafterweise ist jeder Betätigungshebel so dimensioniert, dass beim Umklappen des einen Schenkels 5 des Betätigungshebels ein Exzenterpunkt überschritten wird, wodurch ein unerwünschtes selbsttätiges Aufspringen des Betätigungshebels 3 vermieden wird (Selbsthemmung in Schließposition).

Die Funktion der Verriegelungsmechanismen gemäß der Fig. 1 lässt sich wie folgt beschreiben:
Um den Containerdeckel mit der Containerwanne zu verriegeln werden die beiden an sich gegenüberliegenden Seiten des Sterilcontainers befindlichen Betätigungshebel 3 seriell oder parallel in Schließposition umgeklappt. Dabei werden beide Betätigungsbänder 2 im Bereich Ihrer endseitigen Überlappungen oder teleskopartigen Kopplungsstücke aufeinander zu bewegt, sodass die an dem jeweiligen Betätigungsband jeweils angekoppelten Verriegelungselemente 4 gemeinsam betätigt werden. Sofern der eine Schenkel 5 jedes Betätigungshebels 3 am Containerdeckel oder Containerwanne fest angelenkt ist, wirkt jeder Betätigungshebel unabhängig und separat auf ein Betätigungsband 2. Sofern der eine Schenkel 5 jedes Betätigungshebels 3 jedoch an einem der Betätigungsbänder und der andere Schenkel 6 am jeweils anderen Betätigungsband angelenkt ist, wirkt jeder der zwei Betätigungshebel auf jedes der Betätigungsbänder, an denen jeweils eine Mehrzahl von Verriegelungselementen angekoppelt sind.

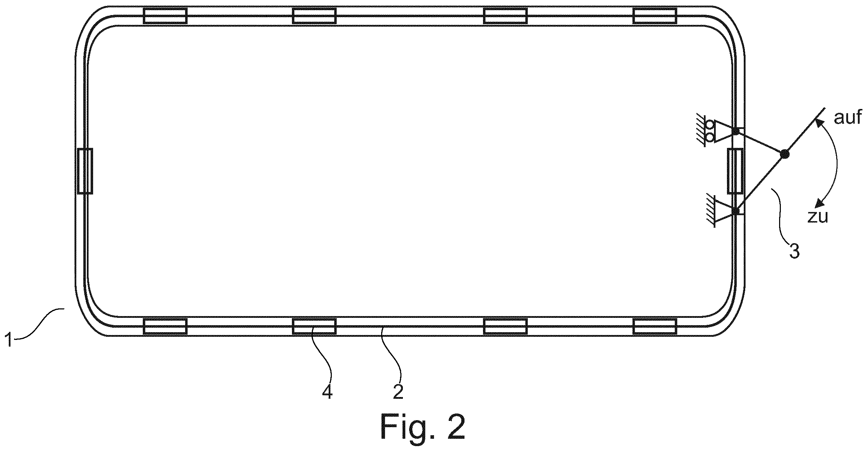

Fig. 2 zeigt einen erfindungsgemäßen Sterilcontainer 1 mit nur einem Betätigungshebel 3 und nur einem Betätigungsband 2. Gleiche Bezugszeichen bezeichnen gleiche oder ähnliche Bauteile wie in Fig. 1.

Auch in dieser Ausführungsform ist das Betätigungsband 2 als ein Flachband oder ein Gestänge ausgebildet, das im Wesentlichen den gesamten Umfang des Containerdeckels umspannt. Da sich das Betätigungsband 2 sowohl beim Öffnungsvorgang als auch beim Schließ- bzw. Verriegelungsvorgang des Sterilcontainers 1 vorzugsweise durchgehend auf Zug befindet, kann alternativ zu dem Gestänge auch ein Seilzug, z.B. aus einem Drahtseil, verwendet werden.

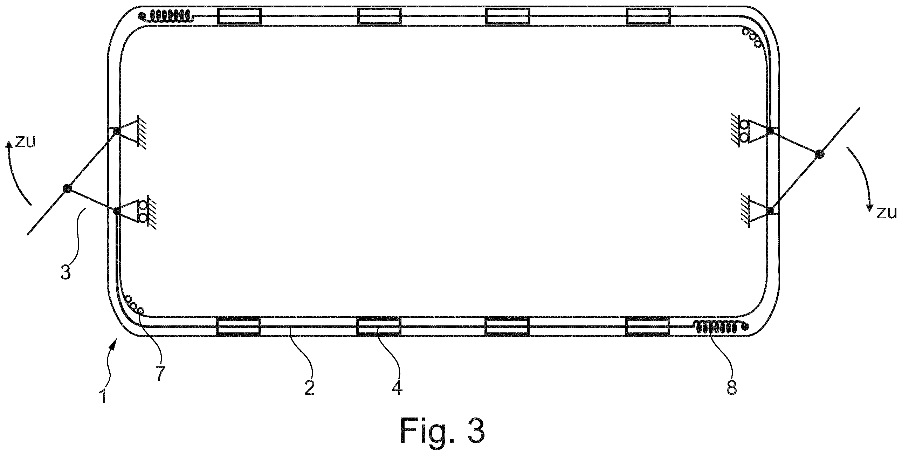

In diesem Ausführungsbeispiel ist ein Schenkel 5 des Betätigungshebels 3 ortsfest mit dem Containerdeckel oder der Containerwanne gekoppelt und der andere Schenkel an einem Ende des Betätigungsbands 2 angelenkt. Das andere Ende des Betätigungsbands 2 ist vorzugsweise über eine am Containerdeckel oder an der Containerwanne befestigte Vorspannfeder (in Fig. 3 nicht weiter dargestellt) auf Zugspannung gehalten.

Wird daher der eine Betätigungshebel 3 in Schließposition umgeklappt verschiebt sich das freie Ende des Betätigungsbands 2 in Richtung hin zu der Vorspannfeder, wobei die Feder weiter gespannt wird. Dabei werden die mit dem Betätigungsband 2 gekoppelten Verriegelungselemente 4 für ein Verriegeln des Containerdeckels an der Containerwanne aktuiert.

Der in Fig. 3 gezeigte Sterilcontainer 1 weist ebenfalls zwei Betätigungshebel 3 und zwei Betätigungsbänder 2 auf. Gleiche Bezugszeichen bezeichnen gleiche oder ähnliche Bauteile wie in den vorhergehenden Figuren.

Bei dieser Ausführungsform sind die beiden Betätigungsbänder 2 separat voneinander ausgebildet und umlaufen jeweils nur knapp die Hälfte des gesamten Umfangs des Deckels des Sterilcontainers 1. Sie bilden somit einen offenen Ring um den Containerdeckel. Jedem Betätigungshebel 3 ist ein Betätigungsband 2 zugeordnet. An ihrem dem jeweiligen Betätigungshebel 3 entgegengesetzten Ende weisen die Betätigungsbänder 2 jeweils eine Zugfeder 8 in Form einer Spiralfeder auf, welche eine Gegenkraft zu der durch Umklappen des jeweiligen Betätigungshebels 3 in eine Schließposition aufgebrachten Kraft bereitstellt, welche die Rückführung der Verriegelungselemente 4 in eine Öffnungsposition unterstützt und das Spiel zwischen Hebel und Betätigungsband sowie zwischen Betätigungsband und Verriegelungselemente 4 verringert.

An den Ecken des Deckels des Sterilcontainers 1 sind die Betätigungsbänder 2 zudem über Rollen 7 gelagert, um die Betätigung der Verriegelungselemente 4 über das jeweilige Betätigungsband 2 zu erleichtern.

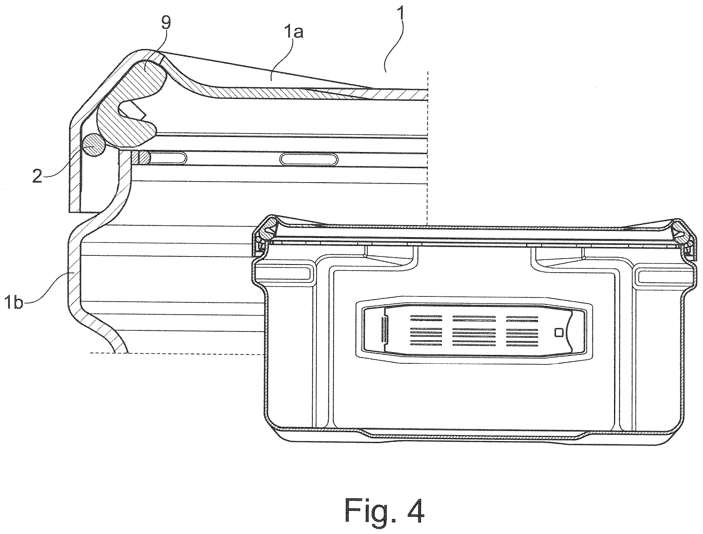

Fig. 4 zeigt eine Querschnittsansicht eines erfindungsgemäßen Sterilcontainers 1, auf welcher die Anordnung des Betätigungsbands 2 zu dem Deckel 1a des Sterilcontainers und einer Dichtung 9 des Sterilcontainers 1 ersichtlich ist. Der gesamte Verschlussmechanismus befindet sich außerhalb der Dichtung 9 und ragt an keiner Stelle in das Innere des Sterilcontainers 1. Die Dichtung 9 ist zwischen dem Deckel 1a und der Containerwanne 1b des Sterilcontainers 1 angeordnet. Während des Schließvorgangs des Sterilcontainers 1 wird der Deckel 1a mittels drehbarer Riegel, Exzenter oder schiefer Ebenen als Beispiele für die zuvor genannten Verriegelungselemente 4 auf die Containerwanne 1b gezogen. Die Dichtung 9 stellt hierbei den elastischen Anteil. Das Betätigungsband 2 ist bei dieser Ausführungsform beispielsweise als Gestänge und / oder als Seilzug ausgebildet und verläuft in einem durch die Dichtung 9 und Wandung des Deckels 1a ausgebildetem Hohlraum. In anderen Worten liegt also die Dichtung 9 auf dem Rand der Containerwanne 1b des Sterilcontainers 1 auf und befindet sich somit zwischen der Containerwanne 1b und dem Deckel 1a des Sterilcontainers 1. Das Betätigungsband 2 verläuft außerhalb der Containerwanne 1b (an der Außenseite) in dem durch den dachartig über die Containerwanne 1a vorstehenden Containerdeckel 1b abgeschirmten Bereich.

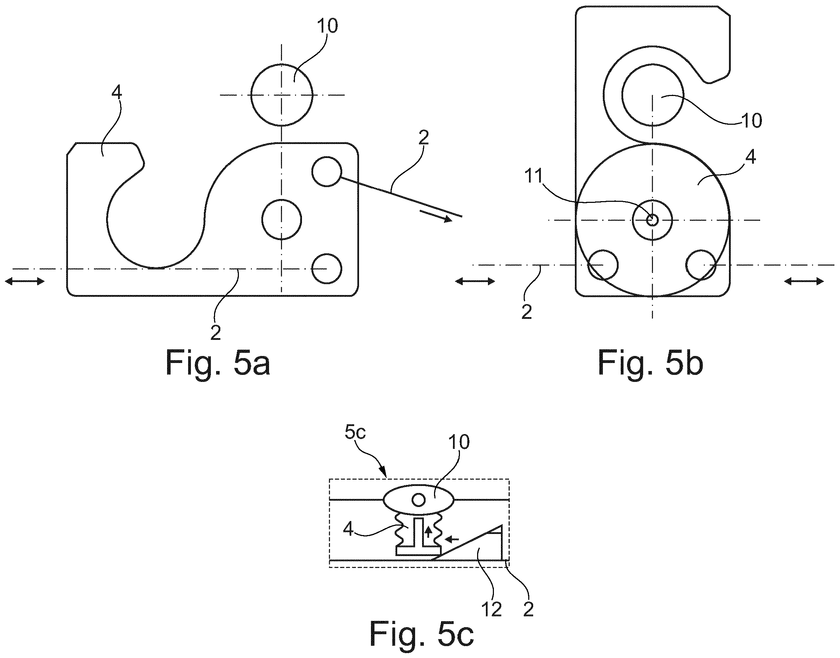

Die Fign. 5a bis 5b zeigen ein Verriegelungselement 4 in Form eines schwenkbar am Containerdeckel oder der Containerwanne schwenkbar angelenkten, haken- oder klauenförmigen Schwenkelements, welches mittels des Betätigungsbands 2 aus einer Öffnungsposition (Fig. 5a, der Sterilcontainer ist geöffnet bzw. unverriegelt) in eine Verriegelungsposition (Fig. 5b, der Sterilcontainer ist geschlossen bzw. verriegelt) verschwenkt wird. In dieser Figur ist das Betätigungsband 2 vorzugsweise ein Seilzug. Bei einer Betätigung des Seilzugs 2 wird das Verriegelungselement in Form des hakenförmigen Schwenkelements um ca. 90 ° aus einer Entriegelungsposition (siehe Fig. 5a) verschwenkt, bis es sich in der Verriegelungsposition (siehe Fig. 5b) im Eingriff befindet mit einem Gegenstück 10, welches in dieser Ausführungsform als Stift ausgebildet ist. Das Verriegelungselement 4 ist in dieser Ausführungsform um einen Schwenkpunkt 11 drehbar gelagert.

Alternativ und / oder zusätzlich kann der Sterilcontainer 1 auch mindestens ein Verriegelungselement 4 in Form eines Verriegelungsstifts aufweisen, wie es in Fig. 5c gezeigt ist. Ein derartiges Verriegelungselement 4 besteht im Wesentlichen aus einem Stift, welcher auf einer Plattform oder Halterung axialverschiebbar montiert ist. An dem Betätigungsband 2 ist eine keilförmige Kulisse oder Rampe 12 vorgesehen. Wird das Betätigungsband 2 betätigt, so verschiebt sich die keilförmige Kulisse 12 entlang der Longitudinalrichtung des Betätigungsbands 2 und unter die Plattform/Halterung des Verriegelungselements/Stifts 4, wodurch der Verriegelungsstift in seine Achsrichtung verschoben wird, welche im Wesentlichen rechtwinklig zu der Longitudinalrichtung des Betätigungsbands 2 verläuft, bis der Verriegelungsstift in ein Gegenstück 10 in Form einer Öse oder Aussparung an der Containerwanne oder am Containerdeckel eingeführt ist (Verriegelungsposition). Dabei ist es vorteilhaft, wenn ein solcher Verriegelungsstift federvorgespannt ist, um selbsttätig in eine Entriegelungsposition zurückzufahren, wenn der Betätigungshebel wieder aufgeklappt wird.

Die vorliegende Erfindung betrifft zusammenfassend einen Sterilcontainer mit einer Mehrzahl von über mindestens ein Betätigungsband durch mindestens einen gemeinsamen Betätigungshebel betätigbaren Verriegelungselementen. Dies ermöglicht sicher eine aktive Kondensatausleitung bei kontrolliertem Druckausgleich.

## Patentansprüche

1. Sterilcontainer mit einer Containerwanne und einem Containerdeckel oder Tür, umfassend eine Mehrzahl von in Containerdeckel-Umfangsrichtung voneinander beabstandeten, an der Containerwanne oder am Containerdeckel beweglich montierten Verriegelungselementen (4), die mit einem gemeinsamen Betätigungsband (2) im Eingriff sind/bringbar sind, das hierfür daran montierte oder ausgebildete Eingriffsabschnitte/Eingriffsglieder hat und das an einen gemeinsamen Betätigungshebel (3) angeschlossen ist, durch den in zentraler Weise die Mehrzahl von Verriegelungselementen (4) über das gemeinsame Betätigungsband (2) betätigbar sind, um die an der Containerwanne oder am Containerdeckel beweglich montierten Verriegelungselemente (4) in eine Ver-/Entriegelungsposition zu bringen, wobei der Betätigungshebel (3) mit jeweils einem daran angeschlossenen Betätigungsband (2) sowie den damit in Wirkeingriff bringbaren/stehenden Verriegelungselementen (4) einen Verriegelungsmechanismus bildet, **dadurch gekennzeichnet, dass** der Betätigungshebel (3) dafür angepasst ist, dass der Betätigungshebel (3), wenn er in eine Verriegelungsposition gebracht wird, einen Exzenterpunkt überschreitet, wodurch ein unbeabsichtigtes Aufspringen des Betätigungshebels (3) verhindert wird.

2. Sterilcontainer nach Anspruch 1, **dadurch gekennzeichnet, dass** zwei dieser Verriegelungsmechanismen vorgesehen sind, die in Serie geschaltet den Umfang des Containerdeckels umgeben, vorzugsweise derart, dass die beiden Betätigungshebel (3) an sich gegenüberliegenden Seiten des Sterilcontainers (1) zu liegen kommen, um gemeinsam eine Zweihandbedienung zum Entriegeln und Verriegeln des Sterilcontainers (1) zu ermöglichen.

3. Sterilcontainer nach einem der vorhergehenden Ansprüche, **dadurch gekennzeichnet, dass** an dem dem Betätigungshebel (3) entgegengesetzten Ende des Betätigungsbands (2) ein elastisches Spannelement vorgesehen ist, das mit dem Betätigungsband (2) und dem Containerdeckel oder der Containerwanne verbunden ist, um das Betätigungsband (2) und somit die damit in Wirkeingriff bringbaren/stehenden Verriegelungselemente (4) in Entriegelungsposition vorzuspannen.

4. Sterilcontainer nach einem der vorhergehenden Ansprüche, **dadurch gekennzeichnet, dass** die Verriegelungselemente (4) haken- oder klauenförmige Schwenkelemente, Verriegelungsstifte oder Verriegelungspilze sind.

5. Sterilcontainer nach einem der vorhergehenden Ansprüche, **dadurch gekennzeichnet, dass** das Betätigungsband (2) die Kontur des Containerdeckels des Sterilcontainers (1) im Wesentlichen nachzeichnet.

6. Sterilcontainer nach einem der vorhergehenden Ansprüche, **dadurch gekennzeichnet, dass** das Betätigungsband (2) im Wesentlichen den gesamten Umfang des Containerdeckels des Sterilcontainers (1) umläuft.

7. Sterilcontainer nach einem der Ansprüche 1 bis 5, **dadurch gekennzeichnet, dass** mehrere Betätigungsbänder (2) vorgesehen sind, welche den Umfang des Containerdeckels des Sterilcontainers (1) jeweils für einen definierten Abschnitt umlaufen.

8. Sterilcontainer nach einem der Ansprüche 1 bis 5, **dadurch gekennzeichnet, dass** das Betätigungsband (2) vorzugsweise an den Ecken des Containerdeckels über Lagerungselemente gelagert und / oder geführt ist.

9. Sterilcontainer nach einem der vorhergehenden Ansprüche, **dadurch gekennzeichnet, dass** das Betätigungsband (2) biegeflexibel ist.

10. Sterilcontainer nach einem der vorhergehenden Ansprüche, **dadurch gekennzeichnet, dass** das Betätigungsband (2) ausschließlich für die Betätigung der mit dem Betätigungsband (2) in Wirkeingriff bringbaren/stehenden Verriegelungselemente (4) angepasst ist.

11. Sterilcontainer nach einem der vorhergehenden Ansprüche, **dadurch gekennzeichnet, dass** das Betätigungsband (2) an dem Containerdeckel oder der Containerwanne unlösbar gehalten ist.

12. Sterilcontainer nach einem der vorhergehenden Ansprüche, **dadurch gekennzeichnet, dass** die mit dem Betätigungsband (2) in Wirkeingriff bringbaren/stehenden Verriegelungselemente (4) Verriegelungsglieder aufweisen, welche an der Containerwanne oder dem Containerdeckel verschiebbar und / oder verschwenkbar gelagert sind und welche zur Bewegungsbetätigung mit den Eingriffsabschnitten/Eingriffsgliedern am Betätigungsband (2) in Wirkeingriff stehen/bringbar sind.

## Claims

1. A sterile container comprising a container trough and a container cover or door, comprising a plurality of locking elements (4) spaced apart from each other in the circumferential direction of the container cover and movably mounted on the container trough or on the container cover which are engaged/can be engaged with a common operating strip (2) that comprises engaging portions/engaging members mounted or formed on the same for this purpose and that is connected to a common operating lever (3) by which the plurality of locking elements (4) can be centrally operated via the common operating strip (2) so as to move the locking elements (4) movably mounted on the container trough or on the container cover into a locking/unlocking position, wherein the operating lever (3) with an operating strip (2) connected thereto and the locking elements (4) to be brought/being in operative engagement therewith forms a locking mechanism, **characterized in that** the operating lever (3) is adapted to exceed, when being moved to a locking position, an eccentric point, thus preventing the operating lever (3) from inadvertently springing open.

2. The sterile container according to claim 1, **characterized in that** two of said locking mechanisms are provided which, when connected in series, surround the periphery of the container cover preferably in such manner that the two operating levers (3) will be located on opposite sides of the sterile container (1) so as to jointly enable a two-handed operation for unlocking and locking the sterile container (1).

3. The sterile container according to any one of the preceding claims, **characterized in that** at the end of the operating strip (2) that is opposed to the operating lever (3) an elastic tensioning element is provided which is connected to the operating strip (2) and to the container cover or the container trough so as to pre-stress the operating strip (2) and thus the locking elements (4) adapted to be/being operatively engaged therewith into the unlocking position.

4. The sterile container according to any one of the preceding claims, **characterized in that** the locking elements (4) are hook-shaped or claw-shaped pivoting elements, locking pins or mushroom-shaped locking bolts.

5. The sterile container according to any one of the preceding claims, **characterized in that** the operating strip (2) substantially follows the contour of the container cover of the sterile container (1).

6. The sterile container according to any one of the preceding claims, **characterized in that** the operating strip (2) runs along substantially the entire periphery of the container cover of the sterile container (1).

7. The sterile container according to one of claims 1 to 5, **characterized in that** a plurality of operating strips (2) is provided each of which runs along the periphery of the container cover of the sterile container (1) for a respective defined section.

8. The sterile container according to any one of the claims 1 to 5, **characterized in that** the operating strip (2) is preferably supported and/or guided at the corners of the container cover via bearing elements.

9. The sterile container according to any one of the preceding claims, **characterized in that** the operating strip (2) is flexible.

10. The sterile container according to any one of the preceding claims, **characterized in that** the operating strip (2) is adapted exclusively for operating the locking elements (4) which can be/are operatively engaged with the operating strip (2).

11. The sterile container according to any one of the preceding claims, **characterized in that** the operating strip (2) is held to be non-releasable on the container cover or the container trough.

12. The sterile container according to any one of the preceding claims, **characterized in that** the locking elements (4) which can be/are operatively engaged with the operating strip (2) include locking members that are shiftably and/or pivotably supported on the container trough or the container cover and that, for actuating movement, can be/are operatively engaged with the engaging portions/engaging members at the operating strip (2).

## Revendications

1. Récipient stérile avec une cuve de récipient et un couvercle de récipient ou une porte, comprenant une pluralité d'éléments de verrouillage (4) espacés les uns des autres dans la direction périphérique de couvercle de récipient, montés de manière mobile sur la cuve de récipient ou sur le couvercle de récipient, qui sont/peuvent être amenés en prise avec une bande d'actionnement (2) commune, qui possède à cet effet des parties de mise en prise/organes de mise en prise montés ou réalisés sur celle-ci et qui est raccordée à un levier d'actionnement (3) commun, par lequel la pluralité d'éléments de verrouillage (4) peuvent être actionnés de manière centrale par l'intermédiaire de la bande d'actionnement (2) commune, afin d'amener les éléments de verrouillage (4) montés de manière mobile sur la cuve de récipient ou sur le couvercle de récipient dans une position de verrouillage/déverrouillage, dans lequel le levier d'actionnement (3) forme avec respectivement une bande d'actionnement (2) raccordée à celui-ci ainsi que les éléments de verrouillage (4) pouvant être amenés/se tenant en prise active avec celle-ci un mécanisme de verrouillage, **caractérisé en ce que** le levier d'actionnement (3) est adapté pour que le levier d'actionnement (3), lorsqu'il est amené dans une position de verrouillage, dépasse un point excentrique, ce qui a pour effet qu'un relâchement involontaire du levier d'actionnement (3) est empêché.

2. Récipient stérile selon la revendication 1, **caractérisé en ce que** deux de ces mécanismes de verrouillage sont prévus, qui de manière montée en série entourent la périphérie du couvercle de récipient, de préférence de telle sorte que les deux leviers d'actionnement (3) viennent se situer sur les faces opposées du récipient stérile (1), afin de permettre conjointement une manipulation à deux mains pour le déverrouillage et le verrouillage du récipient stérile (1).

3. Récipient stérile selon l'une quelconque des revendications précédentes, **caractérisé en ce qu'**un élément de serrage élastique, qui est relié à la bande d'actionnement (2) et au couvercle de récipient ou à la cuve de récipient, afin de précontraindre la bande d'actionnement (2) et avec elle les éléments de verrouillage (4) pouvant être amenés/se tenant en prise active avec celle-ci dans la position de déverrouillage, est prévu à l'extrémité de la bande d'actionnement (2) opposée au levier d'actionnement (3).

4. Récipient stérile selon l'une quelconque des revendications précédentes, **caractérisé en ce que** les éléments de verrouillage (4) sont des éléments pivotants en forme de crochets ou de griffes, des tiges de verrouillage ou des champignons de verrouillage.

5. Récipient stérile selon l'une quelconque des revendications précédentes, **caractérisé en ce que** la bande d'actionnement (2) reproduit sensiblement le contour du couvercle de récipient du récipient stérile (1).

6. Récipient stérile selon l'une quelconque des revendications précédentes, **caractérisé en ce que** la bande d'actionnement (2) fait sensiblement le tour de toute la périphérie du couvercle de récipient du récipient stérile (1).

7. Récipient stérile selon l'une quelconque des revendications 1 à 5, **caractérisé en ce que** plusieurs bandes d'actionnement (2) sont prévues, lesquelles font le tour de la périphérie du couvercle de récipient du récipient stérile (1) respectivement pour une partie définie.

8. Récipient stérile selon l'une quelconque des revendications 1 à 5, **caractérisé en ce que** la bande d'actionnement (2) est montée et/ou guidée de préférence sur les coins du couvercle de récipient par l'intermédiaire d'éléments de montage.

9. Récipient stérile selon l'une quelconque des revendications précédentes, **caractérisé en ce que** la bande d'actionnement (2) est souple en flexion.

10. Récipient stérile selon l'une quelconque des revendications précédentes, **caractérisé en ce que** la bande d'actionnement (2) est adaptée exclusivement pour l'actionnement des élément de verrouillage (4) pouvant être amenés/se tenant en prise active avec la bande d'actionnement (2).

11. Récipient stérile selon l'une quelconque des revendications précédentes, **caractérisé en ce que** la bande d'actionnement (2) est retenue de manière non détachable sur le couvercle de récipient ou la cuve de récipient.

12. Récipient stérile selon l'une quelconque des revendications précédentes, **caractérisé en ce que** les éléments de verrouillage (4) pouvant être amenés/se tenant en prise active avec la bande d'actionnement (2) présentent des organes de verrouillage, lesquels sont montés de manière coulissante et/ou pivotante sur la cuve de récipient ou le couvercle de récipient et lesquels se tiennent/peuvent être amenés en prise active avec les parties de mise en prise/organes de mise en prise sur la bande d'actionnement (2) pour l'actionnement du mouvement.
